# EUROPEAN PATENT APPLICATION

(11) **EP 4 242 638 A2**
(43) Date of publication of application: **13.09.2023**
(21) Application number: 21857905.0
(22) Date of filing: 15.10.2021
(51) Int. Cl.: G01N 21/55, G01N 33/68

(54) **DEVICE AND METHOD FOR MEASURING THE LEVEL OF BIOMARKERS AND PATHOGENS IN SUBSTANCES**

(71) Applicant: Cor Sync Desenvolvimento de Sistemas Ltda, 81280330 Curitiba (BR)
(72) Inventor: DE MACEDO QUEIXADA, Raul, 80710-240 Curitiba (BR); GARCIA DE SÁ, Paulo Henrique, 81280-330. Curitiba (BR)
(74) Representative: Clarke, Modet y Cía., S.L.
(86) International application number: PCT/IB2021/059537
(87) International publication number: WO 2022/038586

(57) **Abstract**

The present invention relates to a small device for measuring the concentration level of troponin and other biomarkers and pathogens in substances using an optical method based on surface plasmon resonance, pertaining to the technical field of diagnosis/identification of biomarkers for detecting cardiac diseases.

## Description

### Field of the Invention

The present invention relates to a small device for measuring the concentration level of troponin and other biomarkers and pathogens in substances by means of an optical method based on surface plasmon resonance, which is intended for the technical field of diagnosis/identification of biomarkers for detection of heart diseases. The invention can also be applied to the technical field of diagnosis/identification of biomarkers for the detection of other types of diseases, as well as the identification of pathogens.

### Background of the invention

Acute myocardial infarction (AMI) is one of the leading causes of death in the world, along with the rest of the group of coronary artery diseases, reaching 7.4 million in 2015 (WORLD HEALTH ORGANIZATION, 2017). In 2018, the number of deaths from acute myocardial infarction in Brazil reached 93,272 deaths in 2016 (BRAZILIAN MINISTRY OF HEALTH, 2018).

According to studies carried out in 2000 (ANTMAN *et al.* , 2000; BERTRAND, 2000; KERN; PARASKOS, 2000), the risk stratification protocols used (at the time) should be improved, as there was evidence that about 75% of the admissions of patients presumably with AMI were incorrect. The use of biomarkers has been established as an essential step to support the diagnosis, whenever possible. The use of cardiac troponin (cTn) in its two isoforms, cTnl and cTnT, was reported as the new standard, providing greater sensitivity than CK-MB (ANTMAN *et al.,* 2000; BERTRAND, 2000; KERN; PARASKOS, 2000) .

Even so, the electrocardiogram (ECG) remains the main tool for diagnosing myocardial infarction. New, or presumably new, significant ST-segment T wave (ST-T) changes, new left bundle branch of His (LBBB or BRE) blocks, and development of pathological Q waves are listed as indicators of myocardial infarction. The term ST-segment elevation acute myocardial infarction (STEMI or SSST AMI) is used when, in the presence of characteristic chest pain, these pathological waves are identifiable on the patient's ECG.

When there are no identifiable pathological waves, the term non-ST segment elevation acute myocardial infarction (NSTEMI or SSST AMI) is used, as well as the more generic term, non-ST segment elevation acute coronary heart disease (NSTE-ACS), which includes a wider spectrum of diseases (THYGESEN *et al.,* 2012). The recommended management of confirmed cases of STEMI includes the immediate referral of the patient to the hemodynamics laboratory, where cardiac catheterization will be performed and, when indicated, coronary angioplasty in order to unclog the occluded artery and restore local blood flow (coronary reperfusion).

When angiography is not available, thrombolytic treatment and monitoring of reperfusion criteria, defined as decreased ST-segment elevation, pain relief, and early drop in cardiac troponin level are recommended. Referral to a health institution with structure to perform cardiac catheterization is mandatory, since in cases where drug treatment with thrombolytics does not show improvement in reperfusion criteria, the patient should be referred for catheterization as soon as possible. For cases where thrombolytic treatment promotes reperfusion, the treatment window is extended to 24 hours (IBANEZ *et al.*, 2017). For NSTEMI cases, the identification of pathological waves on the ECG becomes more complex and the measurement of the cTn level becomes a fundamental tool for the diagnosis, together with the clinical history, risk factors and patient profile evaluated in risk scores such as TIMI, GRACE and HEART (ROFFI *et al.*, 2016).

The performance of laboratory tests to measure the level of troponin by methods such as the enzyme-linked immunosorbent assay (ELISA) has an incubation time of about 1 hour, in addition to the transport time to the laboratory and the test preparation time, thus having a high response time (turn around time - TAT), conflicting with the need to immediately start the treatment of AMI (BHATNAGAR D, PALIT S , SINGH MP, KAUR I, 2016; PERIYAKARUPPAN *et al.,* 2013). The Brazilian guideline, with the objective of promoting the initiation of treatment in the shortest possible time, recommends that "Markers should not be used for diagnostic purposes in patients with SCACST (Acute Coronary Syndrome With ST-segment elevation) and the results should not be waited for to start treating patients. Its greatest value in patients with SCACST is prognostic" (PIEGAS *et al.,* 2015).

Concern about the length of care is shown to be supported by evidence indicating that the rapid diagnosis of coronary heart disease and acute myocardial infarction can reduce morbidity and mortality rates (NEUMANN *et al.*, 2017). However, about 36% of patients with suspected STEMI who undergo catheterization examination have normal coronary angiography, which usually indicates that there is no infarction of atherosclerotic cause, indicating the existence of unnecessary costs for the health system (MCCABE). *et al.,* 2012).

Approximately 70% of the cases of acute coronary diseases are NSTE-ACS in the United States of America, which represents about 540 thousand people per year (AMSTERDAM *et al.,* 2014). According to the "Guideline of the Brazilian Society of Cardiology on the treatment of acute myocardial infarction with ST-segment elevation", the change in the ST segment has a sensitivity of only 45 to 60% for the diagnosis of acute myocardial infarction, and about 50 % of patients have a normal or non-diagnostic ECG (PIEGAS *et al.*, 2015).

The most promising solution to provide measurement of cardiac troponin level with TAT below 1 hour is point of care (PoC) testing. PoC devices are defined as portable devices or desktop devices that perform tests without the need for patient sample preparation, in places such as the emergency care triage post or the doctor's office (BHATNAGAR D, PALIT S , SINGH MP, KAUR I , 2016; HAN *et al.,* 2016; JINJIE; WEI, 2016), making it possible to provide care according to recommendations that facilities that receive patients should have the ability to perform ECG and measure levels of cardiac troponin with immediate referral to the emergency department when an ACS is identified (AMSTERDAM *et al.,* 2014). According to the Brazilian guideline, "Biochemical markers of myocardial injury should be measured in every patient with suspected ACS. Troponins are the biochemical markers of choice." with evidence of level A class I (PIEGAS *et al.,* 2015).

Several devices for the identification and measurement of biomarker levels of cardiac injury or other ischemic injuries have been developed, using different techniques. Patent AU 2012200505 A1 shows a device that transports the sample using active microfluidics to a chamber where an optical analysis is performed (PHAN *et al.*, 2015). Patent US5747274A shows a system that identifies three biomarkers of cardiac injury by linking the biomarkers with their respective antibodies and subsequent color change visible to the human eye (JACKOWSKI, 1998). Patent WO2009047283A2 describes a method of diagnosing ischemic heart disease based on the measurement of biomarkers associated with drug treatment of the condition (HESS; HORSCH; ZDUNEK, 2008).

Patent WO2008089282A2 describes a cardiopulmonary bypass system that monitors the concentration of nitric oxide through antigen-antibody associations (SILVER; MOSTOWFI, 2005). Patent WO2018222783A1 describes a method of assessing brain injury using measurement of cTnl level in conjunction with other biomarkers (MCQUISTON *et al.,* 2018). Patent WO2017011329A1 describes a method, materials and a device for identifying free nucleic acids in samples for the diagnosis of stroke (BARR; GIERSCH; GRANT O'CONNELL, 2016).

Patent US20180100862A1 describes a high sensitivity system and method for analyzing cTnl using fluorescence (GOIX *et al.,* 2018). Patent BR102012003901 A2 describes a device for the detection of cTnT using luminescence (MONTE, ADAMO FERREIRA GOMES DOMADURRO *et al.,* 2012). Patent WO2010127001 A1 describes a biomarker detection system using waveguides and piezoelectric actuators (DUER, REUVEN, 2010). Patent WO2007058872A2 describes an intravenous implantable sensor for detection of stroke, ischemia and infarction biomarkers.

The use of surface plasmon resonance for the construction of biosensors has been growing in the last 20 years and today it is capable of analyzing complex matrices such as blood, saliva and urine. Technological evolution in the area of nanoparticles is one of the protagonists in the feasibility of diagnostic devices based on the surface plasmon resonance phenomenon (HOMOLA, 2003; HOMOLA; PILIARIK, 2006; LIEDBERG; NYLANDER; LUNDSTRÖM, 1995; MARIANI; MINUNNI, 2014).

The use of SPR with the aid of antibody-functionalized gold nanofilm is reported as a viable method for providing accurate and rapid measurements of cardiac troponin I (cTnl) (WU; LI; *et al.,* 2017) and cardiac troponin T (cTnT) (PAWULA; ALTINTAS; TOTHILL, 2016). Patent WO2011106322 A1 describes an imaging device that uses surface plasmon resonance to image of very small particles (FINE; MACAULAY; KREPLAK, 2014).

Patent WO2013084074A2 shows a troponin I measurement system using spectroscopy and metallic nanoparticles diluted in an aqueous solution to measure the localized surface plasmon resonance, which is altered according to the presence of the biomarker (FARIAS *et al.,* 2012). Patent US20040186359A1 describes a surface plasmon resonance system that uses optical fibers to conduct light from the light source to the reaction surface and from there to a spectrophotometer that measures the wavelength, which can be used in vitro or in vivo. For the in vivo system, the existence of two measurement regions is also described, one of the regions without the binding elements and the other region with the binding elements (BEAUDOIN *et al.*, 2002).

Patent WO1992021973A1 presents the method and sensors for using surface plasmon resonance to detect infarction biomarkers in a sample carried by one or more flow cells according to the binding of analytes in specific antibodies, as well as their subsequent removal by the circulation of a regenerative liquid (PEDERSEN; STALBERG, 1991). Patent JPH11211725A describes a method of binding antibodies, antigens and metallic film using hydrophobic binding or electrostatic binding for the construction of a sensor for surface plasmon resonance used for the measurement of infarction biomarkers (NAGATA, RYOHEINAKAGAWA *et al.*, 1998). Patent US7212692B2 describes a sensor using waveguides and surface plasmon resonance and detection by interferometry or detection of electromagnetic wave amplitude (MING YAN, 2007).

The SPR method is a viable solution as demonstrated in several studies and patents, achieving low coefficients of variation and low test time, even with the challenge of identifying cTn molecules in complex substances such as blood. The cTn molecules have a diameter smaller than 10 nm (approximately 4.5 nm for cTnl and 6.3 nm for cTnT), and can be detected with an evanescent field penetration at about 100 nm for an infrared laser on a 5 nm AuNP film, and three-dimensional layer of ligands up to 80 nm (including anti-cTn). The difficulty of differentiating between target molecules and other molecules present in complex substances (bulk effect) can be overcome with the use of several techniques, such as the use of high specificity anti-cTn, ligand arrangements, use of markers, among others.

Given the equipment that performs the measurement of biomarkers in complex substances, such as blood, plasma, saliva, urine, among others, one of the main challenges is the aforementioned bulk effect. In the surface plasmon resonance method, the variation in the concentration of different molecules in the body of each individual can increase the susceptibility of the system to errors that generate loss of sensitivity and specificity.

The present invention presents the use of differential measurement of the same sample in two or more distinct regions on the face of the biosensor, where the first presents a functionalized nanofilm with ligand elements and antibodies of the biomarker of interest, while the second presents a non-functionalized nanofilm, performing the measurement of the resonance angle of the same substance with the presence of the biomarker or pathogen of interest and without its presence. The sample can receive diluted markers to increase the specificity and sensitivity of the test.

The present invention also presents a gain in sensitivity and specificity, eliminating the need for microfluidic circulation of the sample. Reducing the complexity of the equipment allows for its miniaturization and cost reduction, making its use viable in places where it would previously be unfeasible, such as triage rooms and emergency care in hospitals, clinics, ambulances, among others. On the other hand, the present invention, due to the lack of sample circulation, may not present optimal results when applied to the analysis of the dynamics of binding between antigens and antibodies as a function of time.

### Brief description of drawings

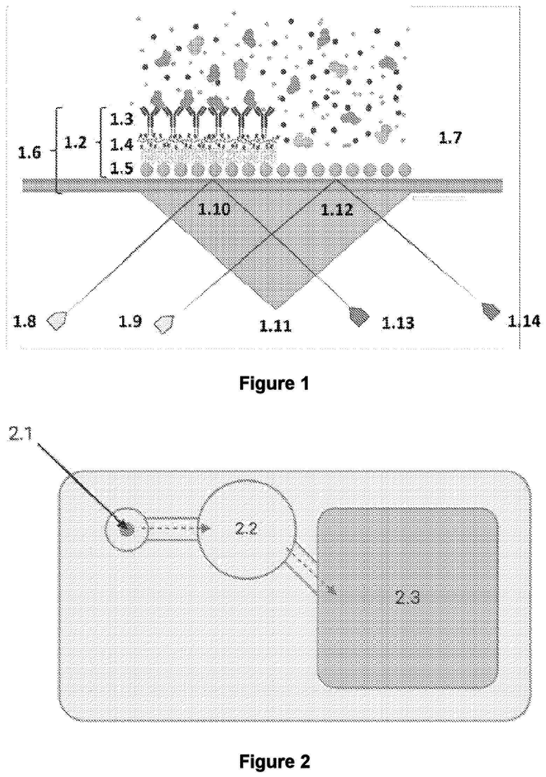

- Figure 1 presents a simplified illustration of the biosensor, its reaction with the sample and the emission, reflection and measurement system of the laser beam passing through the prism where 1.1 represents the Sample; 1.2 represents the Functionalized Part; 1.3 represents Antibodies; 1.4 represents the Ligands; 1.5 represents the Gold Nanofilm; 1.6 represents the Biosensor; 1.7 represents the non-functionalized Part; 1.8 represents the Prism.
- Figure 2 presents the schematic drawing of the sample substance deposition process, where 2.1 represents the sample insertion point, 2.2 a chamber, and 2.3 the biosensor.

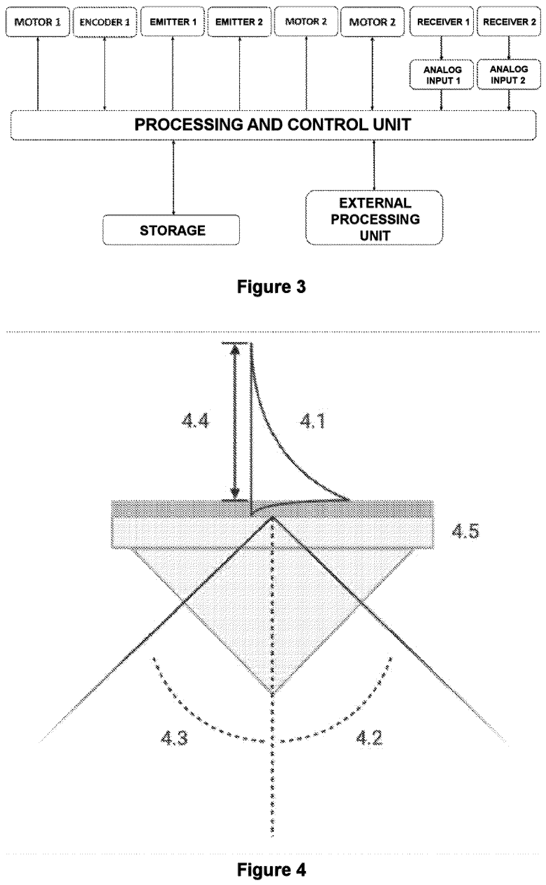

- Figure 3 shows the block diagram of data traffic and control signals of one of the possible configurations of the present invention.
- Figure 4 shows the angle of incidence of the laser beam 4.2 and the reading angle of the receiver element 4.3. Other elements present in the figure are: 4.1 representing the evanescent field, 4.4 the penetration of the evanescent field, and 4.5 a plate of material with a known index of refraction.

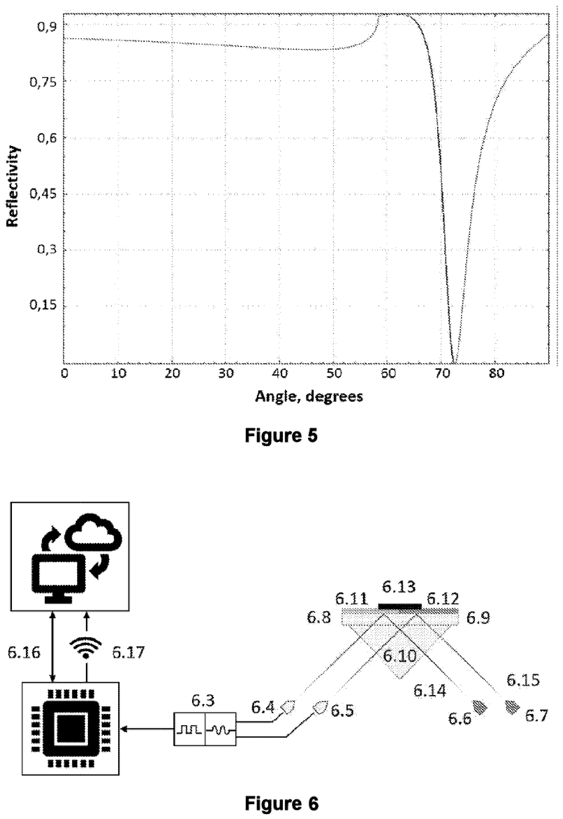

- Figure 5 shows the graphic plot of the reflected relative intensity value (Reflectivity) read by the receiver element and the incidence/reading angle (Angle, degrees) of a scanning cycle.
- Figure 6 shows an overview of the device, where 6.1 represents Sources external to the system; 6.2 the Control and Processing Unit; 6.3 the Analog to Digital Converter; 6.4 and 6.5 Receivers elements; 6.6 and 6.7 Emitters; 6.8 Gold nanofilm; 6.9 Plate of material with known refractive index; 6.10 Prism; 6.11 Functionalized part of the biosensor; 6.12 Non-functionalized part of the biosensor; 6.13 Sample / substance to be analyzed; 6.14 and 6.15 Laser beams; 6.16 Cable for data transmission; 6.17 Representation for wireless data transmission.

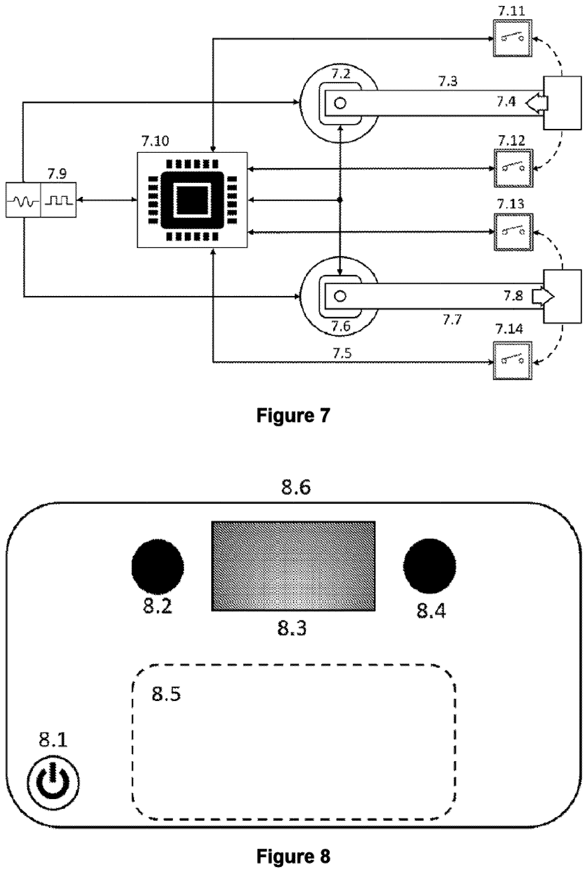

- Figure 7 shows a representation of the mechanical mechanisms of the invention, where 7.1 and 7.5 represent the Motor; 7.2 and 7.6 the Encoder; 7.3 and 7.7 the Arm for movement; 7.4 the Emitter; 7.8 the Receiver Element; 7.9 represents the Interpretation and conversion of signals from the encoder; 7.10 a Control and Processing Unit; and 7.11, 7.12, 7.13 and 7.14 the Power switches.
- Figure 8 shows a frontal representation of the external part of the invention, where 8.1 represents the on/off button, 8.2 button for opening the sensor compartment, 8.3 numerical display, 8.4 button for starting and stopping the test, 8.5 approximate position of the internal components, 8.6 is the encapsulation.

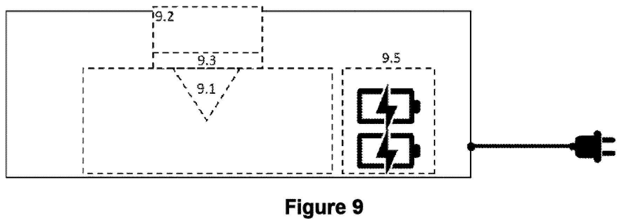

- Figure 9 shows a side representation of the external part of the invention, where 9.1 is the prism, 9.2 the sensor compartment, 9.3 the sensor, 9.4 the encapsulation, 9.5 batteries, 9.6 the cable to be connected to the electrical grid.

### Description of the invention

The present invention relates to a device and method that performs the measurement of the level of troponin and other biomarkers or pathogens in substances such as, and not restricted to, blood, plasma, saliva and urine, as well as its method of functioning and operation.

A biomarker is understood as a molecule consistently modified or present in abnormal concentrations as a result of a disease or clinical condition, which are explored by the discipline of proteomics, responsible for analyzing the protein composition of biological fluids, tissues and other specimens. The definition of a biomarker for a given disease is a complex process that involves several disciplines such as proteomics, genomics and pathophysiology, in addition to tests until it is accepted and adopted as a parameter for the diagnosis of a given disease.

Troponin is defined as the most specific and sensitive biomarker for the diagnosis of acute myocardial infarction with cardiac injury. Troponin is a complex of three units: troponin I, T and C, located on the actin filament together with tropomyosin, being responsible for the calcium-mediated regulation of skeletal and cardiac muscle contraction. It can also be found in unbound in the cytosolic pool of myocytes. Cardiac troponin C is shared with slow-twitch skeletal muscles and is therefore not indicated for use as a biomarker of acute myocardial infarction. The cardiac troponin I isoform has a dissimilarity of 42 and 45% in relation to other isoforms. Troponin T, on the other hand, has four isoforms, only one of which is common in adult cardiac tissue, but showing a smaller dissimilarity in relation to other isoforms. Even so, tests of equivalent specificity between troponin I and C are reported. Both isoforms are released acutely during the event of cardiac injury.

Pathogens are defined as disease-causing microorganisms such as bacteria, viruses, protozoa, fungi and helminths. Pathogens are studied by the discipline of microbiology and its subdisciplines such as pathology, pathophysiology and virology. The identification of the presence of a pathogen in the organism of a host can be used for the diagnosis of diseases caused by infectious agents.

In a main aspect, the present invention addresses the technical problem of diagnosing acute myocardial infarction in emergency care, and can be applied to the diagnosis of other diseases.

The present invention comprises a device for optically measuring the effect of surface plasmon resonance by angular modulation in a biosensor that receives a static sample of the substance to be analyzed.

According to the method of the present invention, the surface plasmon resonance effect is understood as the excitation of surface plasmon polaritons, which in turn are defined as electromagnetic waves propagated at the interface between a metallic material and a dielectric material, or more generally, a material of negative permittivity and a material of positive permittivity. The excitation can be performed by means of photons or electrons, the former being chosen for the present invention. The incidence of a beam of light on the metal-dielectric interface, at a specific angle (4.2) above the condition of total internal reflection, generates the resonance of electrons in the direction parallel to the interface. The field generated by the excitation is an evanescent field (4.1), penetrating the dielectric medium with exponential decay intensity. The penetration (4.4) of the evanescent field, as well as the resonance angle, is dependent on the materials applied at the interface, the thickness of the metal, the wavelength of the light beam, among other factors.

In the present invention, the use of the surface plasmon resonance phenomenon for biosensors is based on the alteration of the refractive index of the dielectric medium by the presence of biomarkers or pathogens immobilized by antibodies in the penetration range of the evanescent field, thus generating a measurable angular modulation using the mechanisms and methods described.

The biosensor (1.6) consists of a plate of material with a known refractive index, with gold nanofilm or another material of similar permittivity (1.5). The biosensor has one or more parts of its face functionalized (1.2) with ligands (1.4) and antibodies (1.3) able to be bound to the surface and perform the antigen-antibody binding of the biomarker of interest. The biosensor also has one or more non-functionalized parts of its face (1.7), which serves as a basis for the differential measurement, measuring only the bulk density of the sample (1.1) without antigen-antibody reaction.

The plate of material with known index of refraction has the function of coupling the photons in the surface plasmon polaritons matching their vectors and moments. This element is necessary because free photons do not have the same scattering factor as surface plasmon polaritons at certain frequencies, thus having their moments misaligned. The plate material can be BK-7 glass, SF11 glass or other material with a known refractive index (4.5). The plate thickness must also be known, and the angle of incidence must be compensated for according to the thickness and refractive index. The use of different materials can influence the resonance angle, sensitivity and specificity of the system.

The gold nanofilm (1.5), or another material of similar permittivity, has the function of a material of negative permittivity at the metal-dielectric interface, making the presence of surface plasmons possible. The material must have the real part of its negative dielectric constant and of magnitude greater than that of the dielectric, making it possible to use gold, silver, aluminum, copper, among others. The conductive material, together with the wavelength, can influence the resonance angle, specificity and sensitivity of the system.

According to the present invention, the functionalized parts (1.2) with ligands (1.4) may or may not have a covalently bound carboxymethyl dextran hydrogel layer, a polyethylene glycol monolayer, a thiol monolayer, or another component with the function of increasing the surface association capacity by covering the metallic film uniformly, enabling immobilization with strong bonds between the surface and the ligands and antibodies necessary to capture the biomarkers or pathogens present in the substance. In order for the surface plasmon resonance effect to be effectively applied, the layer must be uniform and of a known refractive index, with a thickness such that, together with the ligands, antibodies and target molecules, it does not exceed the penetration depth (4.4) of the evanescent field (4.1).

According to the present invention, the ligands (1.4) are defined as a functionalization layer containing proteins, enzymes and antibodies able to be bound to the surface, and carrying out the antigen-antibody binding of the biomarker or pathogen of interest is understood as the immobilization of agents capable of capturing the molecule of interest on the surface of the biosensor within the penetration region (4.4) of the evanescent field (4.1). The functionalization can be done through the activation of carboxylic groups of the antibody and deposition on the sensor with a thiourea monolayer, with the antibodies (1.3) binding through the amines present in the thiourea. Another possible configuration is the use of a carboxymethyl dextran layer in a covalently bound hydrogel functionalized with streptavidin and biotinylated antibodies. The antibody can be an anti-troponin of the analyzed isoform or the antibody of the biomarker or pathogen to be analyzed, or even a protein to identify the immunological response existing in the sample.

According to the method of the present invention, to carry out the test, the sample (6.13) must be deposited on the surface of the biosensor (6.8 and 6.9), which in turn is placed on a glass prism (6.10) of known refractive index. An emitter (6.6) generates a laser beam of a suitable wavelength for the application (6.14), and is incident on one of the faces of the prism, reaching the functionalized part of the biosensor (6.12) and being reflected to a receiver element (6.5) connected to the analog input of a control and processing unit (6.2), which performs the conversion of the analog value into a numerical value (6.3), being able to store and send the numerical value that represents the intensity of the reflected laser beam, in addition to other data that may be relevant. Another emitter positioned displaced in relation to the first one (6.6) emits a laser beam of the same wavelength (6.14), which is then incident on the same face of the prism, in turn reaching the non-functionalized part of the biosensor (6.11) and being reflected to another receiver element (6.4), read in the same way as the previous one. Both results are stored and sent. Parameters can be received from external sources (6.1).

According to the method of the present invention, the sample of the substance to be analyzed is deposited on the surface of the biosensor and remains static, without the need for microfluidic circulation.

According to the method of the present invention, during the process of deposition of the substance on the surface of the biosensor, it passes through one or more chambers (2.2) where the sample can be mixed with reagents of different functions, such as anticoagulant, buffering agent (buffer) , markers for sandwich methodology, among others. The presence of anticoagulant reagents such as heparin, lithium heparin and EDTA has the function of keeping the viscosity low, when the substance to be analyzed is blood or plasma, consequently promoting good molecular diffusion, thus accelerating the antigen-antibody association.

The buffering agent has the function of controlling the pH of the solution through chemical bonds, preventing unwanted reactions. The sandwich methodology can be applied in the present invention to increase the sensitivity and specificity of the test, having gold nanoparticles or other material diluted in a neutral solution and bound to antibodies specific for binding to epitopes of a particular region of the biomarker molecule or pathogen to be analyzed. The binding of these nanoparticles with the analyte increases its density, thus increasing the magnitude of the change in the shrinkage index when the analyte is captured on the surface of the biosensor. Additionally binding with gold nanoparticles or other materials generates an additional resonance by LSPR. The presence of reagents in the chamber may or may not exist as a result of the specific application of the present invention.

After the deposition of the substance, which is inserted at the insertion point (2.1), it will pass through the chamber (2.2) where mixing of the sample with reagents can occur. The dashed line arrows show the direction of sample entry, which after being deposited remains static on the biosensor (2.3).

According to the present invention, the prism (6.10) is defined as a component with the function of coupling the photons with the surface plasmon polaritons, matching their vectors and moments. The prism must have a known index of refraction similar to that of the biosensor, which is necessary because free photons do not have the same scattering factor as surface plasmon polaritons at certain frequencies, thus having their moments misaligned. The use of different vitreous materials can influence the resonance angle, sensitivity and specificity of the system.

According to the present invention, the emitter (6.6 and 6.7) is understood to be responsible for generating a laser beam of a wavelength suitable for application as a light-emitting diode or lamp capable of emitting a beam of light of wavelength defined and suitable for the excitation of surface plasmon polaritons at the metal-dielectric interface, this wavelength being commonly in the visible or near infrared spectrum, with a length of 850 nm, but other spectra may be used according to the used conductive material.

The emitted light is collimated with an exit angle small enough so that there is no undesired detection of the beam in receiver elements to which it is not directed, this angle being commonly in the order of magnitude of 1° to 6°, but may be different according to the positioning of the emitters and receiver elements. The emitted light has known polarization so that, when it reaches the metal-dielectric interface, its vector and momentum are aligned with that of the surface plasmon polaritons.

The emitter has a controlled and known emission intensity, allowing its compensation to avoid the insertion of associated errors. The limitation of wavelength, collimation, polarization, intensity control and any other control of the emitted light can be carried out by the emitter component or components external to it, which together can be considered as an emitter system. There are one or more emitters in each construction of the present invention.

The receiver element (6.4 and 6.5) is defined as a component measuring the intensity of the laser beam reflected by the biosensor after interacting with the electromagnetic field of the surface plasmon resonance. The receiver element has a controlled detection band so that only the wavelength of the beam of interest is detected, ensuring that the system does not suffer interference from waves originating from the environment and not relevant to the test. The detection angle (4.3) of the receiver element is also controlled, being collimated and specific in the direction of the reflected beam, thus avoiding the detection of interfering waves, which in the infrared range can be generated not only by light sources, but also by of heat.

The circuit associated with the receiver element (6.2 and 6.3) has voltage and current control with the detection stabilization function, promoting shielding against variations in the electrical grid to which it is connected. The receiver element can be designed by using photodiodes, phototransistors, LDR, CMOS sensors, among others. There are one or more receptor elements in each construct of the present invention.

The analog input (6.3) is understood as a continuous or variable voltage level reading channel in relation to the reference plane, and may or may not have a signal conditioning circuit such as passive or active filters, voltage divider, Wheatstone's bridge, Kelvin's Bridge, Maxwell's Bridge, Anderson's Bridge, Hay's Bridge, Owen's Bridge, Campbell's Bridge, Schering's Bridge, operational amplifier, instrumentation amplifier, level translators, among others. The conditioning circuit has the function of filtering, adjusting the reference, resolution, among other parameters, and is considered as part of the analog input system. The analog-to-digital converter, responsible for converting the voltage, current, impedance or frequency value into a discrete digital value, is part of the analog input and may or may not be integrated into the control and processing unit.

According to the present invention, the control and processing unit (6.2) is understood as the processing and control unit of the system, where the algorithm or other type of control and processing programming is executed.

The control and processing unit performs the open or closed loop control of the laser beam emitters (6.6 and 6.7), reading and interpretation of the receiver elements (6.4 and 6.5), closed loop control of positioning motors and mathematical conversion calculation of parameters read in concentration values of the biomarker or pathogen in the analyzed substance (6.13), in addition to the storage, sending and receiving of information. The control and processing unit may have analog and digital inputs and outputs for direct control of the functions for which it is intended or use peripherals to adjust the level of voltage, current, impedance, frequency or any other relevant characteristic for the performance of the functions for the which it is intended. The control unit can be conceived through the use of microcontrollers (single or multi-core), microprocessors (single or multicore), FPGA, GPU, ASIC, ULA, through combinational logic, or multi-architectures (which include the use of two or more previously described architectures).

According to the present invention, the storage of values is understood as the process of storing data in a volatile or non-volatile memory, which can be internal (6.2) or external (6.1) to the control and processing unit, as long as it can be accessed with permission to write, change and read by the same, regardless of the time interval between the write, change and read actions.

According to the present invention, sending values is understood as the process of sending data to other processing or storage units through a wired connection (6.16) or not (6.17). Examples of processing or storage units are computers, cell phones, local or cloud servers, among others.

The data can be sent in real time or in packets containing values referring to a period.

Data may include, but is not limited to: initialization message (handshake), time the initialization message was sent (timestamp), device serial number, calibration values, calibration date, current activity (status), value numeric that represents the intensity of the reflected laser beam, angle of incidence of the laser, numerical result of the concentration of molecules of the biomarker or pathogen, time of collection of the parameter, time of sending the parameter, confirmation of receipt of messages (acknowledgement) and code of message integrity conference (checksum). Data are organized according to a proprietary communication protocol and compressed and sent according to the communication protocol applicable to the layer used. The sending medium can be, but is not limited to: TTL, RS232, modbus, ethernet, Wi-Fi and GSM.

According to the present invention, receiving parameters is the process of receiving data from other processing or storage units through a wired or non-wired connection. Examples of processing or storage units are computers, cell phones, local or cloud servers, among others. The data can be received in real time or in packets containing values referring to a period. Data may include, but are not limited to: initialization message (handshake), time of sending the initialization message (timestamp), acknowledgment of receipt of messages (acknowledgement), time of sending the parameter and integrity check code of messages (checksum). Data are organized according to a proprietary communication protocol and compressed and sent according to the communication protocol applicable to the layer used. The sending medium can be, but is not limited to: TTL, RS232, modbus, ethernet, Wi-Fi and GSM. Figure 3 presents a simplified schematic diagram of data traffic and control signals for one of the possible configurations of the present invention.

According to the present invention, the emitters (7.4) are mounted on an arm (7.3) fixed to the shaft of a motor (7.1) with angular movement control. The receiver elements (7.8) are mounted on another arm (7.7) fixed to another motor (7.5) with angular movement control positioned on the opposite side of the prism. The motors change the laser incidence angle and the reading angle of the receiver element, and the procedure of emitting the laser beam and analog reading of the receiver element is repeated with each change in angle. The values of all readings (scanning) are plotted against the reading angle to form a surface plasmon resonance plot (5) allowing the analysis of the resonance angle for the functionalized and non-functionalized part of the biosensor.

The motor with angular motion control is defined as a system composed of an angular motion motor and an angular positioning sensor. The motor can be of the stepper motor, servomotor or other type with controlled angular increment capacity and electric drive. The angular positioning sensor is a rotary encoder (7.2 and 7.6) that produces electrical pulses at each angular increment performed by the motor shaft, which can be either incremental or absolute. The angular increment needed to generate an encoder pulse is defined by its resolution. The pulses can be interpreted and converted to numerical values (7.9) representing the current angle of the motor in a dedicated processing unit or in the control and processing unit (7.10). When starting to work, the motor with angular motion control performs an initialization procedure, where it is moved to known angles, indicated by binary activation keys (7.11, 7.12, 7.13, and 7.14). The control and processing unit receives the value change from the switches and associates the drive position with the known angle. The motor with angular motion control is closed-loop controlled by an algorithm based on encoder reading and angular increment actuation. There are one or more motors with angular motion control in each construction of the present invention. The angular increment of each motor changes the laser incidence angle or the reading angle of the receiver element.

According to the present invention, the laser incidence angle (4.2 and 4.3) is defined as the angle formed by the imaginary line from the center of the emitted laser beam, centered with the emitter, and the imaginary line perpendicular to the face of the biosensor and passing through the lower edge of the prism. The reading angle of the receiver element is defined as the angle formed by the imaginary line from the center of the detection field of the receiver element, centered with the receiver element, and the imaginary line perpendicular to the face of the biosensor and passing through the lower edge of the prism. The laser incidence and reading angles of the receiver element are closed-loop controlled by the motor with angular motion control. Figure 4 presents an illustration of the laser incidence angle and the reading angle of the receiver element.

According to the present invention, the scanning procedure is understood as a set of control and reading actions necessary for the analysis of the resonance angle for each functionalized part (6.11) and each non-functionalized part of the biosensor (6.12). Among these actions, the following sequence stands out: angular positioning of the laser emitter (6.6 and 6.7) and of the receiver element (6.4 and 6.5), reading (6.3) and storage (6.1 and 6.2) of the laser incidence angle and of the reading angle of the receiver element (4.2 and 4.3 in Figure 4), emission of the laser beam in the functionalized part of the biosensor (6.4), reading by the receiver element (6.4) of the intensity of the laser beam reflected by the functionalized part of the biosensor after interacting with the electromagnetic field of surface plasmon resonance, emission of the laser beam in the non-functionalized part of the biosensor (6.12), reading by the receiver element (6.5) of the intensity of the laser beam reflected by the non-functionalized part of the biosensor after interacting with the electromagnetic field of the surface plasmon resonance, data storage and sending. The described sequence is repeated until the entire angular range of interest has been covered. The angular range of interest normally ranges from 45° to 90°, and may vary according to several parameters, such as the wavelength of the emitted beam, the material of the prism and the biosensor plate, the material of negative permittivity of the biosensor, the substance to be analyzed, among others. The sequence can be repeated with negative angular increment to check and compensate for hysteresis effects, being considered part of the scanning.

According to the present invention, the plot is understood as the compilation and organized display of the reading values of the receiver element in relation to the angle of incidence of the laser beam. Compilation and organized display can be performed graphically as shown in Figure 2 or in table, matrix or another format. The plotting function in the system is the subsequent identification of the resonance angle of each functionalized and non-functionalized part of the biosensor and data analysis and treatment. Figure 5 represents the graphical plot of a scanning cycle.

According to the present invention, the angle with the lowest reading value of the receiver element is the resonance angle, which is the value of greatest interest in the scanning process. The resonance angle is the angle of incidence of the laser beam where there is the smallest reading value by the receiver element of the intensity of the laser beam reflected by the biosensor after interacting with the electromagnetic field of the surface plasmon resonance.

The resonance angles read will be applied as variables in a series of mathematical equations that relate them to known constants, such as: the refractive index of air, prism, gold nanofilm, polymer, protein, enzyme, antibody and antigen; evanescent field strength generated by resonance; and changing the resonance angle; providing numerical results of the density of the sample in the functionalized and non-functionalized parts, and then a subtraction between them is performed, to then obtain the numerical value of the concentration of molecules of the biomarker or pathogen of interest in the analyzed sample.

According to the present invention, the device in which the described parts are encapsulated has dimensions small enough for the device to be used on a table or bench, or carried by a person. The encapsulation (8.6) is done by one or more components that form an enclosure around the described parts (8.5). The enclosure must be constructed of conductive material connected to the neutral or grounding plane, in order to shield the device against electromagnetic interference, as well as to prevent internally generated electromagnetic emissions from affecting the environment. The conductive housing may or may not have finishing parts around the same.

The user's operation interface must have: an on or off state selector with a state indicator or not (8.1); a test start and stop button (8.4); and a numerical display for viewing the test result and other information (8.3). Additionally, the interface may or may not have a button to open the sensor insertion compartment (8.2). The interface components can be mechanical, electromechanical, electronic or virtual, attached to or external to the device.

The insertion of the sensor (9.3) containing the sample to be analyzed is done manually or automatically in the device (9.4), provided that there are means to optimize the contact of the sensor surface with the prism surface (9.1). The insertion can be direct or have an opening mechanism for the sensor insertion compartment (9.2), activated by a button on the interface (8.2) or manual opening.

The device is powered by electrical energy from an external or internal source. In the case of power supply from external sources, the device is connected to the electrical grid by means of a cable (9.6), and the electrical current is conditioned for use by an internal or external circuit, which may contain an alternating current to direct current converter, interference filters, short circuit, overcurrent and overvoltage detection system, among others. In the case of internal power supply, the device has one or more disposable or rechargeable battery packs (9.6), which may or may not pass through a conditioning circuit for use.

## Claims

1. A device for measuring the concentration level of troponin and other biomarkers or pathogens, **characterized in that** it contains biosensors, reaction chambers, prism, emitters, receivers, analog inputs, control and processing units and motors with angular control.

2. The device for measuring the concentration level of troponin and other biomarkers or pathogens according to claim 1, **characterized in that** the biosensor (1.6) consists of a plate of material with a known retraction index (4.5), with a gold nanofilm or other material of similar permittivity (1.5) with one or more functionalized parts (1.2) and one or more non-functionalized parts (1.7), intended for differential measurement by a surface plasmon resonance method.

3. The device for measuring the concentration level of troponin and other biomarkers or pathogens according to claim 2, **characterized in that** the functionalized parts (1.2) contain ligand elements (1.4) and antibodies (1.3) for measuring the antigen-antibody reaction with the biomarker or pathogen of interest and the non-functionalized parts (1.7) have the surface of the nanofilm exposed to measure the bulk density (mass) of the sample (1.1).

4. The device for measuring the concentration level of troponin and other biomarkers or pathogens according to claim 1, **characterized in that** it contains one or more chambers (2.2) through which the sample passes during its insertion process in the biosensor and where its mixing with reagents can occur.

5. The device for measuring the concentration level of troponin and other biomarkers or pathogens according to claim 1, **characterized in that** the emitters (6.6 and 6.7) emit one or more laser beams that pass through the prism (6.10) and reach the biosensor, being reflected and read by the receivers (6.4 and 6.5), which emit signals processed in the analog inputs (6.3) and interpreted by the control and processing units (6.2), with the angle of incidence of the emitter (4.3) and the reading angle of the receiver (4.2) being controlled by motors with angular control (7.1 and 7.5).

6. A method for measuring the concentration level of troponin and other biomarkers or pathogens, **characterized in that** it performs a differential optical measurement of the resonance effect of surface plasmons by angular modulation in a biosensor with a static sample/substance, following the steps of sample insertion, passage through the chambers, antigen-antibody reaction, scanning, plotting and obtaining the numerical result.

7. The method for measuring the concentration level of troponin and other biomarkers or pathogens according to claim 6, **characterized in that** the differential measurement is carried out through scanning and identification of the resonance angles of the sample applied to the functionalized (1.2) and non-functionalized (1.7) parts, its subsequent application in equations, and subtraction of the results to obtain the numerical result of the concentration of molecules of the biomarker or pathogen of interest in the analyzed sample.

8. The method for measuring the concentration level of troponin and other biomarkers or pathogens according to claim 7, **characterized in that** scanning and identifying the resonance angles of the sample applied to the functionalized parts are dependent on the antigen-antibody reaction that occurs in the range of penetration of the evanescent field (4.4) in the functionalized parts and **in that** scanning and identifying the resonance angles of the sample applied to the non-functionalized parts are dependent on the bulk density of the sample present in the evanescent field penetration range in the non-functionalized parts.

9. The method for measuring the concentration level of troponin and other biomarkers or pathogens according to claim 6, **characterized in that** the sample/substance to be analyzed is inserted at the insertion point (2.1), passes through one or more chambers (2.2 ) and is finally deposited on the biosensor (2.3), where it remains static during the antigen-antibody reaction, scanning, plotting and obtaining the numerical result, without the need for microfluidic circulation.

10. The method for measuring the concentration level of troponin and other biomarkers or pathogens according to claim 6, **characterized in that** the scanning consists of the sequence of angular positioning, reading and storage of the incidence angle of the emitter and the reading angle of the receiver (7.9), followed by the emission of the beam (6.14) in a functionalized part of the biosensor (6.11), reading by the receiver (6.4) of the intensity of the beam reflected by the functionalized part of the biosensor after interacting with the electromagnetic field of the surface plasmon resonance, emission of the beam (6.15) in a non-functionalized part of the biosensor (6.12), reading by the receiver (6.5) of the intensity of the laser beam reflected by the non-functionalized part of the biosensor after interacting with the electromagnetic field of the surface plasmon resonance, storage and sending of the reading data (6.16 and 6.17).

11. The method for measuring the concentration level of troponin and other biomarkers or pathogens according to claim 10, **characterized in that** the scanning sequence is repeated for both functionalized and non-functionalized parts existing in the biosensor, throughout the angular range of interest and can be performed with angular increment or decrement, with the function of collecting numerical values, which are graphically plotted (5), or not, to identify the resonance angle of each functionalized and non-functionalized part of the biosensor, necessary for the application of the method of differential measurement.
